# EUROPÄISCHE PATENTSCHRIFT

(11) **EP 2 222 338 B1**
(45) Veröffentlichungstag und Bekanntmachung des Hinweises auf die Patenterteilung: **21.12.2016**
(21) Anmeldenummer: 08852496.2
(22) Anmeldetag: 12.11.2008
(51) Int. Cl.: A61K 47/02, A61K 47/10, A61K 47/44

(54) **STABILISIERUNG ÖLIGER SUSPENSIONEN ENTHALTEND HYDROPHOBE KIESELSÄUREN**
STABILISATION OF OILY SUSPENSIONS CONTAINING HYDROPHOBIC SILICIC ACIDS
STABILISATION DE SUSPENSIONS HUILEUSES CONTENANT DES ACIDES SILICIQUES HYDROPHOBES

(30) Priorität: 19.11.2007 DE 102007055341
(43) Veröffentlichungstag der Anmeldung: 01.09.2010
(73) Patentinhaber: Bayer Intellectual Property GmbH, 40789 Monheim (DE)
(72) Erfinder: HEEP, Iris, 50859 Köln (DE); HAMANN, Hans-Juergen, 41539 Dormagen (DE); KÖLLING, Sabine, 51375 Leverkusen (DE); DAUBE, Gert, 51766 Engelskirchen (DE); STEFFENS, Klaus-Jürgen, 53359 Rheinbach (DE); DANIELS, Rolf, 38226 Salzgitter (DE)
(74) Vertreter: BIP Patents
(86) Internationale Anmeldenummer: PCT/EP2008/009526
(87) Internationale Veröffentlichungsnummer: WO 2009/065514

(56) Entgegenhaltungen:
- EP-A- 0 733 357
- WO-A-2005/018641
- WO-A-2005/025566
- WO-A-2006/061155
- WO-A-2006/061156
- WO-A1-00/01371
- US-A1- 2003 054 043
- DATABASE WPI Week 199217 Thomson Scientific, London, GB; AN 1992-136698 XP002521021 & JP 04 077421 A (EISAI CO LTD) 11. März 1992 (1992-03-11)
- DATABASE WPI Week 199441 Thomson Scientific, London, GB; AN 1994-329916 XP002521022 & JP 06 256178 A (NIPPON OILS & FATS CO LTD) 13. September 1994 (1994-09-13)
- ANONYMOUS: 'Hydrophobic fumed silica - AEROSIL fumed silica', [Online] 01 Januar 2014, Seiten 1 - 2, XP055159809 Gefunden im Internet: <URL:https://www.aerosil.com/product/aerosi l/en/products/hydrophobic-fumed-silica/page s/default.aspx> [gefunden am 2014-12-22]

## Beschreibung

Die Erfindung betrifft die Stabilisierung von fluiden Suspensionen auf Ölbasis enthaltend eine hydrophobe Kieselsäure, sowie Arzneimittel auf Basis solcher Suspensionen.

Ein Arzneimittel auf Basis von Ölen zu formulieren kann verschiedene Vorteile haben, z.B. in der Verträglichkeit für den Patienten oder in der Stabilität des Wirkstoffs. Pharmazeutisch verwendbare Öle haben aber nicht immer die erforderliche Viskosität, um z.B. ein Sedimentieren von Wirkstoffen zu reduzieren oder zu vermeiden. So gibt es bekannte Einsatzstoffe, die die Viskosität von Ölen erhöhen, die meisten davon können zwar in der Kosmetik, nicht aber in der Pharmazie eingesetzt werden. So werden z.B. kolloidale Kieselsäuren zur Verdickung von Ölen für Arzneimittel verwendet. (US 2001-0006671, US 4079131,WO 03-063877)

In WO 2006/008640 werden kolloidalem Siliciumdioxid (eine hydrophile Kieselsäure) nichtionische Emulgatoren zugesetzt, damit der Wirkstoff in Ölen besser benetzt wird.

In WO 03/022254 werden "colloidal silica", hydrophile und hydrophobe Kieselsäuren, mit hydrophilen Polymeren wie z.B. Polyethylenglykol 200 oder Polyvinylalkohol in Ölen versetzt. Die Interaktion der Kieselsäure mit dem Polymer soll zu reduziertem Einsatz der Kieselsäure, höherer Fließgrenze und geringerer Viskosität bei Scherung führen

FR 2790200 benennt ebenfalls hydrophobe Kieselsäuren allerdings explizit speziell ohne den Zusatz von Stabilisatoren.

In WO 2006/061155 und WO 2006/061156 werden nicht nur hydrophile sondern auch hydrophobe Kieselsäuren in Ölen beschrieben. Dabei können auch als allgemeine Einsatzstoffe z.B. nichtionische Tenside oder auch z.B. Polyethylenglykol 200 verwendet werden.

Hydrophile Kieselsäuren haben in öligen Formulierungen den Nachteil, dass sie sehr empfindlich gegenüber Wasser oder Feuchtigkeit sind und bereits Feuchtigkeit aus der Umgebungsluft adsorbieren. Daraus folgt, dass sich die Viskosität der Formulierung erhöht und z.B. die Entnehmbarkeit aus dem Primärpackmittel nicht in dem angegebenen Maße mehr möglich ist. Dies ist insbesondere für Arzneimittel nicht akzeptabel. Werden beispielsweise Einmaldosenbehältnisse aus Kunststoff für diese Formulierungen verwendet, kommt der Effekt der Empfindlichkeit gegenüber Feuchtigkeit besonders zum Tragen, da Kunststoffbehältnisse nicht gegenüber Feuchtigkeit dicht sind und die Formulierung im abgefiillten Behältnis Feuchtigkeit zieht und die Viskosität ansteigen kann. Zudem ist das Verhältnis der Oberfläche der Formulierung zur umgebenden Luft bei Einmaldosenbehältnissen im Vergleich zu Mehrdosenbehältnissen besonders ungünstig.

Durch die Verwendung hydrophober Kieselsäuren zur Stabilisierung von Suspensionen gegen Sedimentation kann diese Empfindlichkeit gegenüber Feuchtigkeit überwunden werden. Ein deutlicher Nachteil der hydrophoben Kieselsäuren ist allerdings die schlechtere Langzeitstabilität bezogen auf die Sedimentationsstabilisierung im Vergleich zu den hydrophilen Kieselsäuren. Die schlechtere Langzeitstabilität ist dadurch gekennzeichnet, dass innerhalb von Wochen oder Monaten die ursprüngliche Viskosität der öligen Formulierungen sinkt, was für Arzneimittel nicht akzeptabel ist, denn dies kann z.B. zu unerwünschten Sedimentationen führen.

Überraschenderweise wurde gefunden, dass die Langzeitstabilität öliger Formulierungen mit hydrophoben Kieselsäuren deutlich durch den Zusatz amphiphiler Verbindungen verbessert wird. Dieser Effekt tritt bei Einsatz hydrophiler Kieselsäuren kaum auf. Dieser Effekt wurde für ölige Suspensionen, die speziell hydrophobe Kieselsäuren enthalten, noch nicht im Stand der Technik beschrieben.

Gegenstand der Erfindung ist daher:

Die Verwendung von amphiphilen Substanzen zur Stabilisierung von fluiden Suspensionen auf Ölbasis enthaltend eine hydrophobe Kieselsäure, in der Silanolgruppen durch Alkylgruppen alkyliert sind, wobei die amphiphile Substanz eine polyoxyethylierte Verbindung ist..

Solche fluiden Suspensionen werden vorzugsweise in Formulierungen für Arzneimittel verwendet.

Daher sind weiterer Gegenstand der Erfindung:

Arzneimittel, enthaltend in einer Ölbasis:
a) einen Wirkstoff
b) eine hydrophobe Kieselsäure
c) eine polyoxyethylierte Verbindung

Auf "Ölbasis" bedeutet, dass die entsprechende Suspension bzw. das entsprechende Arzneimittel eine ölige Grundlage enthält. Als ölige Grundlage können natürliche (tierische oder pflanzliche), synthetische sowie halbsynthetische Öle oder Fette verwendet werden. Dies sind zum Beispiel Soja-, Sonnenblumen-, Baumwollsamen-, Oliven-, Erdnuss-, Distel-, Palm- , Raps-, Kokos-, Maiskeim- oder Rizinusöl. Bevorzugt verwendet werden die Mittelkettigen Triglyceride (Triglyceride mit gesättigten Fettsäuren, bevorzugt der Octan und Decansäure), Propylenglycoldiester der Capryl-/ Caprinsäure, dünnflüssiges Paraffin oder Sesamöl; hiervon besonders bevorzugt verwendet werden die Mittelkettigen Triglyceride und Propylenglycoldiester der Capryl-/ Caprinsäure.

Die ölige Grundlage wird typischerweise in einem Anteil von 99 - 72 Gew.-%, bevorzugt von 99 - 88 Gew.-%, besonders bevorzugt von 97 - 92 Gew.-% bezogen auf die betreffende Suspension oder das fertige Arzneimittel eingesetzt.

Die Suspensionen können unterschiedliche Viskositäten aufweisen, sodass grundsätzlich eine Bandbreite von dünnflüssigen Suspensionen bis zu Pasten denkbar ist. Bevorzugt sind fluide Systeme, welche dünnflüssige und auch dickerflüssige Systeme umfassen, sofern sie unter ihrem Eigengewicht noch fließen. Bevorzugte fluide Systeme haben eine Fließgrenze, d.h. diese Systeme fließen nach Scherung (z.B. durch Schütteln). In vielen Fällen lösen sich die Wirkstoffe nicht oder nicht ausreichend in der fluiden Grundlage, sodass auch der Wirkstoff suspendiert werden muss. Aus diesem Grund werden hydrophobe Kieselsäuren als Verdickungsmittel zur Stabilisierung der Suspension gegen Sedimentation eingesetzt.

Hydrophobe Kieselsäuren sind Kieselsäuren, die von Wasser nicht benetzt werden; das bedeutet, dass sie auf der Wasseroberfläche schwimmen. Ebenfalls in Frage kommen hydrophobisierte Mischoxide aus Siliciumdioxid und Aluminiumoxid, allerdings sind hydrophobe reine Kieselsäuren bevorzugt. Sie werden hergestellt, in dem hydrophile Kieselsäure mit Silanen (Halogensilane, Alkoxysilane, Silazane, Siloxane) versetzt werden. Dabei werden Silanolgruppen durch Alkylgruppen, vorzugsweise mit bis zu 18 Kohlenstoffatomen, besonders bevorzugt mit bis zu 8 Kohlenstoffatomen, ganz besonders bevorzugt mit bis zu 4 Kohlenstoffatomen, insbesondere durch Methylgruppen alkyliert. Beispielsweise werden als Silane bei der Herstellung Hexamethyldisilazan oder bevorzugt Dimethyldichlorsilan verwendet. Die entsprechenden hydrophoben Kieselsäuren können sich ableiten von gefällten, hochdispersen, vorverdichteten oder pyrogenen Kieselsäuren, wobei pyrogene Kieselsäuren bevorzugt sind. Z. B. entsteht durch Umsetzung einer hydrophilen Kieselsäure mit Dimethyldichlorsilan hydrophobes Aerosil, das den Handelsnamen Aerosil® R 972 trägt; dieses hat einen Methylierungsgrad von 66% - 75% (bestimmt durch Titration der Rest-Silanolgruppen).

Das hydrophobe Kieselgel wird in den Formulierungen typischerweise in einem Anteil von 0,1 - 10 Gew.-% eingesetzt, bevorzugt mit 0,5 - 5 Gew.-%, besonders bevorzugt mit 1,5 - 4,0 Gew.-% eingesetzt.

Amphiphile Verbindungen bestehen aus einem polaren (hydrophilen) und einem apolaren (hydrophoben) Teil. Typische Amphiphile sind Fettsäuren, Tenside und Phospholipide. Auch Wirkstoffe können amphiphiler Natur sein. So sind z.B. Chinolone oder auch Fluorchinolone amphiphil. Die Moleküle tragen eine Säuregruppe und eine basische Gruppe. Die Säuregruppe kann deprotoniert vorliegen und trägt dann eine negative Ladung, die basische Gruppe kann protoniert vorliegen und trägt dann eine positive Ladung. Der jeweils geladene Molekülteil ist stark polar und hydrophil, während der Rest des Moleküls weniger polar und damit stärker hydrophob ist.

Im Rahmen dieser Erfindung sind amphiphile Verbindungen polyoxyethylierte Verbindungen. Polyoxyethylierte Verbindungen auch als polyethoxylierte Verbindungen bezeichnet werden z. B. durch Umsetzung mit Ethylenoxid hergestellt. Sie tragen eine oder mehrere aneinanderhängende Einheiten der Formel -[O-CH₂-CH₂]-. Als polyoxyethylierte Verbindungen seien insbesondere genannt:

Nichtionogene amphiphile polyoxyethylierte Verbindungen wie
- Poloxamere, bevorzugt mit Molmassen von 100 bis 5000 g/mol, besonders bevorzugt mit Molmassen von 1000 bis 3500 g/mol. Poloxamer ist der Internationale Freiname für Blockcopolymere aus Ethylenoxid und Methyloxiran,
- Polyoxyethylen-Fettsäureglyceride, auch nicht ionogene Emulgatoren genannt, bevorzugt z. B. Glycerin-Polyethylenglykolricinoleat,
- Polyoxyethylen-Sorbitanfettsäureester, bevorzugt z. B. Polyoxyethylen-20-sorbitan-monooleat,
- Polyoxyethylen-Fettsäuren wie Macrogol-15-Hydroxystearat (= Solutol HS15, erhältlich durch Umsetzung von 15 mol Ethylenoxid und 1 mol 12-Hydroxy-stearinsäure)
- Polyoxyethylen-Fettalkohole wie Hydroxypolyethoxydodecan.

Fettsäure- bzw. Fettalkohol steht insbesondere für die entsprechenden Verbindungen mit mindestens 6 Kohlenstoffatomen und üblicherweise nicht mehr als 30 Kohlenstoffatomen.

Die amphiphile, insbesondere die polyoxyethylierte Verbindung wird in den Formulierungen typischerweise in einem Anteil von 0,001 - 0,15 Gew.-%, bevorzugt mit 0,005 - 0,09 Gew.-% und besonders bevorzugt mit 0,005 -0,08 Gew.%, insbesondere 0,01 - 0,07 Gew.% eingesetzt.

Die erfindungsgemäßen Suspensionen auf Ölbasis werden vorzugsweise in Arzneimitteln eingesetzt. Sie enthalten dann einen oder mehrer pharmazeutische Wirkstoffe.

Genannt seien zum Beispiel Antiinfektiva; dies sind insbesondere antibakteriell wirksame Verbindungen, wie Penicilline, Cephalosporine, Aminoglykoside, Sulfonamide und insbesondere Chinolone.

Chinolone, vorzugsweise Fluorchinolone, sind unter anderem Verbindungen, wie sie in folgenden Dokumenten offenbart sind: US 4 670 444 (Bayer AG), US 4 472 405 (Riker Labs), US 4 730 000 (Abbott), US 4 861 779 (Pfizer), US 4 382 892 (Daiichi), US 4 704 459 (Toyama), als konkrete Beispiele für Chinolone seien Pipemidsäure und Nalidixinsäure genannt; als Beispiele für Fluorchinolone seien genannt: Benofloxacin, Binfloxacin, Cinoxacin, Ciprofloxacin, Danofloxacin, Difloxacin, Enoxacin, Enrofloxacin, Fleroxacin, Ibafloxacin, Levofloxacin, Lomefloxacin, Marbofloxacin, Moxifloxacin, Norfloxacin, Ofloxacin, Orbifloxacin, Pefloxacin, Temafloxacin, Tosufloxacin, Sarafloxacin, Sparfloxacin.

Eine bevorzugte Gruppe von Fluorchinolonen sind die der Formel (I) oder (II): 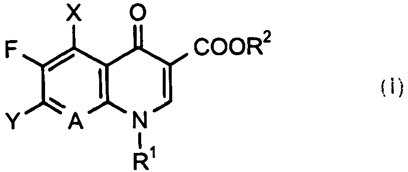 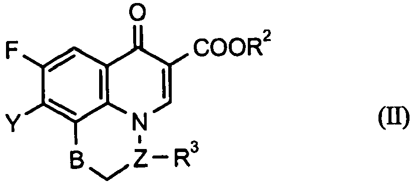 in welchen
- X: für Wasserstoff, Halogen, C₁₋₄-Alkyl, C₁₋₄-Alkoxy, NH₂ steht,
- Y: für Reste der Strukturen 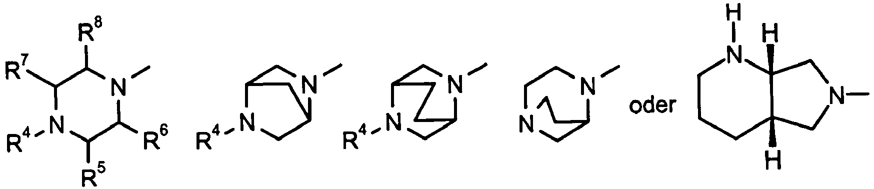
steht, worin
- R⁴: für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
- R⁵: für Wasserstoff, Methyl, Phenyl, Thienyl oder Pyridyl steht,
- R⁶: für Wasserstoff oder C₁₋₄-Alkyl steht,
- R⁷: für Wasserstoff oder C₁₋₄-Alkyl steht,
- R⁸: für Wasserstoff oder C₁₋₄-Alkyl steht,
sowie
- R¹: für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,
- R²: für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
- R³: für Wasserstoff, Methyl oder Ethyl steht und
- A: für Stickstoff, =CH-, =C(Halogen)-, =C(OCH₃)-, =C(CH₃)- oder =C(CN) steht,
- B: für Sauerstoff, gegebenenfalls durch Methyl oder Phenyl substituiertes =NH oder =CH₂ steht,
- Z: für =CH- oder =N- steht,
und deren pharmazeutisch verwendbaren Salze und Hydrate.

Die Verbindungen der Formeln (I) und (II) können in Form ihrer Racemate oder in enantiomeren Formen vorliegen.

Bevorzugt sind Verbindungen der Formel (I),
in welcher
- A: für =CH- oder =C-CN steht,
- R¹: für gegebenenfalls durch Halogen substituiertes C₁-C₃-Alkyl oder Cyclopropyl steht,
- R²: für Wasserstoff oder C₁₋₄-Alkyl steht,
- Y: für Reste der Strukturen 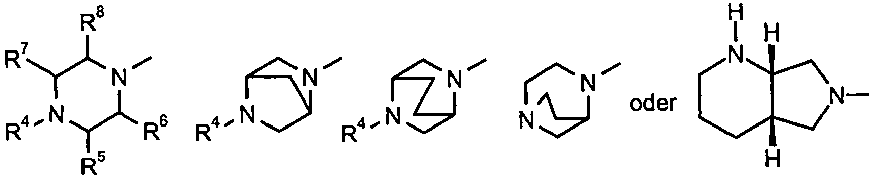
steht, worin
- R⁴: für gegebenenfalls durch Hydroxy substituiertes geradkettiges oder verzweigtes C₁-C₃-Alkyl, Oxalkyl mit 1 bis 4 C-Atomen steht,
- R⁵: für Wasserstoff, Methyl oder Phenyl steht,
- R⁷: für Wasserstoff oder Methyl steht,
- R⁶ und R⁸: für Wasserstoff stehen
und deren pharmazeutisch verwendbaren Hydrate und Salze.

Besonders bevorzugt sind Verbindungen der Formel (I),
in welcher
- A: für =CH- oder =C-CN steht,
- R¹: für Cyclopropyl steht,
- R²: für Wasserstoff, Methyl oder Ethyl steht,
- Y: für Reste der Strukturen 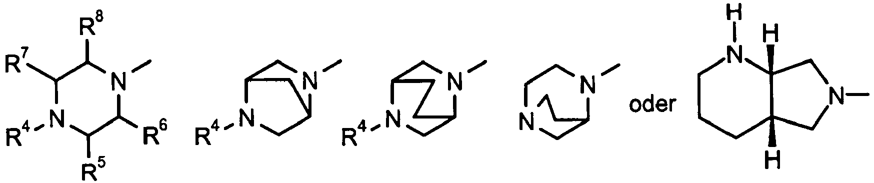
steht, worin
- R⁴: für Methyl, gegebenenfalls durch Hydroxy substituiertes Ethyl steht,
- R⁵: für Wasserstoff oder Methyl steht,
- R⁷: für Wasserstoff oder Methyl steht,
- R⁶ und R⁸: für Wasserstoff stehen
und deren pharmazeutisch verwendbaren Salze und Hydrate.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate der Fluorchinolone selbst als auch die Hydrate von deren Salzen verstanden.

Als besonders bevorzugte Fluorchinolone seien die in WO 97/31001 beschriebenen Verbindungen genannt, insbesondere 8-Cyan-1-cyclopropyl-7-((1S,6S)-2,8-diazabicyclo[4.3.0]nonan-8-yl)-6-fluor-1,4-dihydro-4-oxo-3-chinolincarbonsäure (Pradofloxacin) mit der Formel

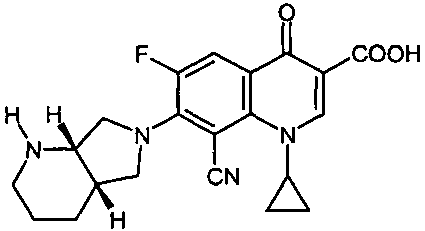

Pradofloxacin wir bevorzugt in Form seines Trihydrats eingesetzt.

Weiterhin besonders bevorzugt eingesetzt wird Enrofloxacin:

### 1-Cyclopropyl-7-(4-ethyl-1-piperazinyl)-6-fluor-1,4-dihydro-4-oxo-3-chinolin-carbonsäure

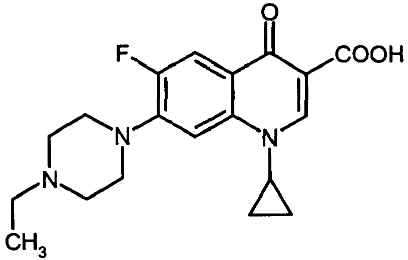

Neben Enrofloxacin und Pradofloxacin seien als bevorzugte Chinolon-Antünfektiva noch Marbofloxacin, Orbifloxacin, Difloxacin, Ofloxacin und Ibafloxacin genannt.

Penicilline sind z.B. Benzylpenicillin, Ampicillin, Amoxicillin, Oxacillin, Piperacillin, Ticarcillin.

Cephalosporine sind z. B.Cefalexin, Cefadroxil, Cefazolin, Cefoxitin, Ceftiofur.

Als Makrolid sei z.B. genannt Erythromycin, Spiramycin, Tylosin, Tilmicosin.

Als Sulfonamide seien z.B. genannt Trimethoprim und Sulfadiazin (bevorzugt in Kombination eingesetzt).

Als Aminoglykoside seien genannt Gentamicin, Kanamycin, Streptomycin, Neomycin und Spektinomycin.

Weiterhin sei als Antibiotikum das Lincosamid Clindamycin genannt.

Weniger bevorzugte Antiinfektiva im Sinne dieser Erfindung leiten sich vom Silber ab, z. B. kolloidales Silber, Silbernitrat oder Silbersulfadiazin. Diese können jedoch in Kombination mit einem der weiter oben beschriebenen Antiinfektiva und/oder gegebenenfalls einem Kortikoid eingesetzt werden.

Das Antiinfektivum wird typischerweise in einem Anteil von 0,001 - 6 Gew.%, bevorzugt 0,01 - 1,0 Gew.%, besonders bevorzugt 0,1 - 0,8 Gew.% bezogen auf das fertige Arzneimittel eingesetzt.

Weiterhin seien als pharmazeutische Wirkstoffe Antimykotika genannt, wie z. B. ein Imidazol oder ein Triazol, Ketokonazol, Enilconazol, Econazol insbesondere z. B. Clotrimazol, Miconazol oder Bifonazol.

Das Antimykotikum wird typischerweise in einem Anteil von 0,01 - 10 Gew.-%, bevorzugt 0,1 - 5 Gew.-%, besonders bevorzugt 0,5 - 2 Gew.-% bezogen auf das fertige Arzneimittel eingesetzt.

Weiterhin kommen als pharmazeutische Wirkstoffe zum Beispiel Kortikoide in Frage. Als Beispiele seien Hydrocortison, Prednisolon, Betamethason, Mometason, Clobetason, Flumethason; bevorzugt Betamethason, Triamcinolon und insbesondere Dexamethason genannt.

Das Kortikoid wird typischerweise in einem Anteil von 0,001 - 2,0 Gew.-%, bevorzugt 0,005 - 0,5 Gew.-%, besonders bevorzugt 0,05 - 0,2 Gew.% bezogen auf das fertige Arzneimittel eingesetzt.

Weiterhin kommen als pharmazeutische Wirkstoffe Triazine in Frage, und zwar insbesondere die Verbindungen der Formeln (I) oder (II): 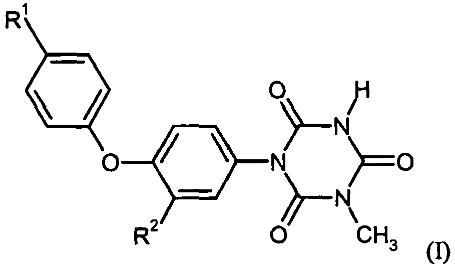 oder 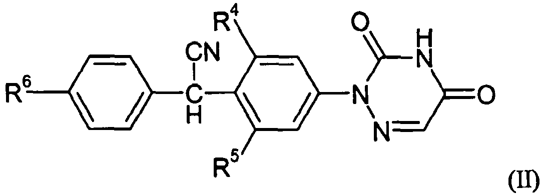 worin
- R¹: für R³-SO₂- oder R³-S- steht,
- R²: für Alkyl, Alkoxy, Halogen oder SO₂N(CH₃)₂ steht und
- R³: für Halogenalkyl steht
- R⁴ und R⁵: unabhängig voneinander für Wasserstoff oder Cl stehen und
- R⁶: für Fluor oder Chlor steht,
sowie ihre physiologisch verträglichen Salze.

Die Triazine sind als Wirkstoffe gegen Coccidien-Infektionen per se gut bekannt, genannt seien die Triazintrione wie z.B. Toltrazuril und Ponazuril sowie die Triazindione wie z.B. Clazuril, Diclazuril und Letrazuril.

Die Triazindione werden durch Formel (II) wiedergegeben:
Clazuril (R⁴ = Cl, R⁵ = H, R⁶ = Cl in Formel (II))
Letrazuril (R⁴ = Cl, R⁵ = Cl, R⁶ = F in Formel (II)) und
Diclazuril (R⁴ = Cl, R⁵ = Cl, R⁶ = Cl in Formel (II)).
Von diesen 1,2,4-Triazindionen ist Diclazuril am meisten bevorzugt.

Erfindungsgemäß besonders bevorzugt sind die Triazintrione der Formel (I) als Wirkstoffe:
R² steht bevorzugt für Alkyl oder Alkoxy mit jeweils bis zu 4 Kohlenstoffatomen, besonders bevorzugt für Methyl, Ethyl, n-Propyl, i-Propyl.
R3 steht bevorzugt für Perfluoralkyl mit 1 bis 3 Kohlenstoffatomen, besonders bevorzugt für Trifluormethyl oder Pentafluorethyl.

Beispiel für besonders bevorzugte Triazintrione der Formel (I) sind:
Toltrazuril (R¹ = R³-S-, R² = CH₃, R³ = CF₃)
Ponazuril (R¹ = R³-SO₂-, R² = CH₃, R³ = CF₃)

Von diesen ist Toltrazuril am meisten bevorzugt.

Bei allen pharmazeutisch wirksamen Bestandteile können - wie oben für die Chinolone ausführlicher erläutert - die entsprechenden pharmazeutisch akzeptablen Salze, Hydrate, Solvate und gegebenenfalls verschiedene Modifikationen verwendet werden.

Optisch aktive Substanzen können in Form ihrer Stereoisomere oder als Stereoisomerengemisch verwendete werden, z.B. als reine oder angereicherte Entantiomere oder als Racemate.

In den erfindungsgemäßen Suspensionen bzw. Arzneimitteln können die Wirkstoffe jeweils einzeln enthalten sein oder in Kombination mit weiteren Wirkstoffen eingesetzt werden.

Gemäß einer bevorzugten Ausführungsform kann die erfindungsgemäße Formulierung so eingestellt werden, dass sie thixotrope Eigenschaften hat, das heißt, sie wird durch Aufschütteln dünnflüssiger und in Ruhe baut sich die Viskosität wieder auf. Dies führt zu einer guten Entnehmbarkeit aus dem Primärpackmittel, sowie zur einer raschen Rekonstitution; vorteilhaft ist das z. B. bei Anwendungen im Gehörgang, damit die applizierte Fomulierung im Ohr verbleibt und nicht z. B. durch Kopfschütteln herausgeschleudert werden kann. Thixotrope Formulierungen werden hergestellt, indem der Formulierungsgrundlage (fluide, ölige Grundlage) ein entsprechendes Additiv zugesetzt wird, sofern die fluide Grundlage nicht schon selbst thixotrop ist. Ein solches Additiv ist üblicherweise ein Suspensionsstabilisator oder Verdickungsmittel wie z.B. die hochdispersen Siliciumdioxide. Das Ausmaß der Thixotropie kann durch Variation der Konzentration gezielt eingestellt werden.

Die Formulierungen können weitere übliche, pharmazeutisch verträgliche Zusatz- und Hilfsstoffe enthalten. Als Beispiele seien genannt
- Weitere Verdickungsmittel sind in der Regel nicht erforderlich, können aber gegebenenfalls eingesetzt werden. Als weitere Verdicker seien z. B. genannt: Cellulosederivate wie Methylcellulose, Hydroxypropylcellulose, Hydroxyethylcellulose, Hydroxypropylmethylcellulose, Carboxymethylcellulose, mikrokristalline Cellulose; Bentonite, Kaolin, Pectin, Stärken, modifizierte Stärke, Wachse, Agar, Paraffine, Gelatine, Alginate, Polyvinylpyrrolidon, Crospovidon, Cetylalkohol, Stearate wie z.B. Magnesiumstearat, Zinkstearat oder Glycerylstearat, gesättigte oder ungesättigte langkettige Fettsäuren (C₈-C₂₄), hochmolekulare Polyethylenglykole (z.B. Polyethylenglykol 2000) und Kieselsäuren.
- Konservierungsstoffe wie zum Beispiel Carbonsäuren (Sorbinsäure, Propionsäure, Benzoesäure, Milchsäure), Phenole (Kresole, p-Hydroxybenzoesäureester wie Methylparaben, Propylparaben etc.), aliphatische Alkohole (Benzylalkohol, Ethanol, Butanol etc.), quartäre Ammoniumverbindungen (Benzalkoniumchlorid, Cetylpyridiniumchlorid)
- Antioxidantien wie zum Beispiel Sulfite (Na-sulfit, Na-metabisulfit), organische Sulfide (Cystin, Cystein, Cysteamin, Methionin, Thioglycerol, Thioglykolsäure, Thiomilchsäure) Phenole (Tocopherole, wie auch Vitamin E und Vitamin-E-TPGS (d-alpha-Tocopherylpolyethylenglykol-1000-succinat)), Butylhydroxyanisol, Butylhydroxytoluol, Gallussäure (Propyl-, Octyl- und Dodecylgallat), organische Säuren (Ascorbinsäure, Citronensäure, Weinsäure, Milchsäure) und deren Salze und Ester.

- Netzmittel oder Emulgatoren wie zum Beispiel Fettsäuresalze, Fettalkylsulfate, Fettalkylsulfonate, lineare Alkylbenzolsulfonate, Fettalkylpolyethylenglykolethersulfate, Fettalkylpolyethylenglykolether, Alkylphenolpolyethylenglykolether, Alkylpolyglykoside, Fettsäure-N-methylglucamide, Polysorbate, Sorbitanfettsäureester, Lecithine und Poloxamere.
- Pharmazeutisch akzeptable Farbstoffe wie zum Beispiel Eisenoxide, Carotinoide, etc.
- Die Formulierungen können auch Kosolventien enthalten, die die Viskosität herabsetzen. Diese werden üblicherweise in Anteilen von 0,1 bis 40 Gew.-%, bevorzugt von 1 bis 10 Gew.% eingesetzt. Als Kosolventien seien beispielsweise genannt: Pharmazeutisch verträgliche Alkohole wie Ethanol oder Benzylalkohol, Dimethylsulfoxid, Ethyllactat, Ethylacetat, Triacetin, N-Methylpyrrolidon, Glycerinformal, Propylencarbonat, Benzylbenzoat, Glycofurol, Dimethylacetamid, 2-Pyrrolidon, Isopropylidenglycerol, Glycerin und Polyethylenglykole. Auch Mischungen der vorstehend genannten Lösungsmittel können als Kosolvenz eingesetzt werden.
- Wasser
- Als Spreitmittel können unter anderem Hexyldodecanol, Decyloleat, Dibutyladipat, Dimethicon, Glycerylricinoleat, Octyldodecanol, Octylstearat, Propylenglykoldipelargonat und bevorzugt Isopropylmyristat oder Isopropylpalmitat eingesetzt werden.
- Penetrationsenhancer (oder Permeationsenhancer) verbessern die transdermale Applikation von Arzneimitteln und sind im Prinzip im Stand der Technik bekannt (siehe z. B. Kapitel 6 von Dermatopharmazie, Wissenschaftliche Verlagsgesellschaft mbH Stuttgart, 2001). Als Beispiele seien spreitende Öle wie Isopropylmyristat, Dipropylenglykolpelargonat, Silikonöle bzw. deren Copolymerisate mit Polyethern, Fettsäureester (z.B. Ölsäureoleylester), Triglyceride, Fettalkohole sowie Linolen genannt. DMSO, N-Methylpyrrolidon, 2-Pyrrolidon, Dipropylenglykolmonomethylether, Octyldodecanol, Oleylmacrogolglyceride oder Propylenglykollaurat können ebenfalls verwendet werden.

Die Formulierungen werden hergestellt, indem die zu lösenden oder zu suspendierenden Wirk-, bzw. Hilfsstoffe in die Grundlage dispergiert werden. Gegebenenfalls wird zur Dispergierung ein Homogenisator oder Hochdruckhomogenisator, eingesetzt. Die Reihenfolge der Zugabe einzelner Bestandteile kann je nach Formulierung variiert werden. Nach Dispergierung aller Formulierungsbestandteile wird die fertige Formulierung zwischengelagert oder direkt in die Primärpackmittel abgefüllt.

Grundsätzlich kommen alle möglichen Primärpackmittel für die erfindungsgemäß3n Suspensionen bzw. Arzneimittel in Frage Gemäß einer bevorzugten Ausführungsform werden Einmaldosenbehältnisse als Primärpackmittel verwendet. Diese werden mit einem Volumen von 0,1-0,5 ml, bevorzugt 0,2-0,4 ml, besonders bevorzugt 0,3-2,0 ml entnehmbarem Inhalt an Formulierung befüllt.

Die erfindungsgemäßen Arzneimittel eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt, und zwar insbesondere bei Säugetieren.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, sowie Vögel wie z.B. Hühner, Gänse, Puten, Enten, Tauben und Straußenvögel. Beispiele für bevorzugte Nutztiere sind Rind, Schaf, Schwein und Huhn.

Zu Labor- und Versuchstieren gehören Hunde, Katzen, Kaninchen und Nagetiere wie Mäuse, Ratten, Meerschweinchen und Goldhamster.

Zu den Hobbytieren gehören Hunde, Katzen, Pferde, Kaninchen, Nagetiere wie Goldhamster, Meerschweinchen, Mäuse, desweiteren Reptilien, Amphibien und Vögel zur Heim- und Zoohaltung.

Bevorzugt werden die erfindungsgemäßen Arzneimittel bei Hobbytieren, und zwar insbesondere bei Hunden und Katzen eingesetzt.

Die Anwendung kann sowohl prophylaktisch als auch therapeutisch erfolgen.

Die hier beschriebenen Arzneimittel eignen sich grundsätzlich für alle möglichen Applikationsarten wie z.B. die dermale, orale, rectale, vaginale oder nasale Applikation. Besonders geeignet sind sie z. B. für die lokale Applikation in die Gehörgänge.

Die beschriebenen Formulierungen eignen sich daher besonders gut zur hygienischen Behandlung von Erkrankungen des Gehörgangs, wie der Otitis externa, bei Hunden und Katzen. Vorzugsweise werden sie dazu in Einmaldosenbehältnisse als Primärpackmitel abgefühlt. Besonders ist hervorzuheben, dass die Formulierung gut reproduzierbar entnommen werden kann. Falls Verdickungsmittel in Suspensions-Formulierungen eingesetzt werden, kann eine Sedimentation der suspendierten Bestandteile in der Regel verhindert werden. Besonders vorteilhaft sind thixotrope Formulierungen, da nach Aufschütteln der Einmaldosenbehältnisse die Formulierung - selbst bei geringen Wirkstoffkonzentrationen - besonders gut reproduzierbar entnommen, die Formulierung durch die Thixotropie und das Einmaldosenbehältnis einfach und hygienisch in das Tierohr appliziert und dennoch nicht zum Beispiel durch das übliche Schütteln des Kopfes herausgeschleudert werden kann. Wünschenswert ist ebenfalls ein gutes Spreitverhalten der Formulierung, da die Formulierung nach Applikation sich gut im Gehörgang verteilen soll.

Arzneimittel zur Applikation im Gehörgang können vorzugsweise Antibiotika, Kortikoide oder Antimykotika enthalten, wie sie weiter oben beschrieben sind. Bevorzugt ist eine Kombination von Antibiotika und Kortikoiden, besonders bevorzugt eine Dreierkombination von Antibiotika, Kortikoiden und Antimykotika. Die oben bei den jeweiligen Wirkstoffgruppen gemachten Angaben zu bevorzugten Ausführungsformen treffen auch auf diesen Anwendungsbereich zu.

Beispielhaft seien als besonders bevorzugte Wirkstoffkombinationen genannt: Pradofloxacin, Clotrimazol und Dexamethason (bevorzugt in Form seines Acetats) sowie Enrofloxacin, Bifonazol und Dexamethason (bevorzugt in Form seines Acetats).

### Bestimmung der Sedimentationskinetik

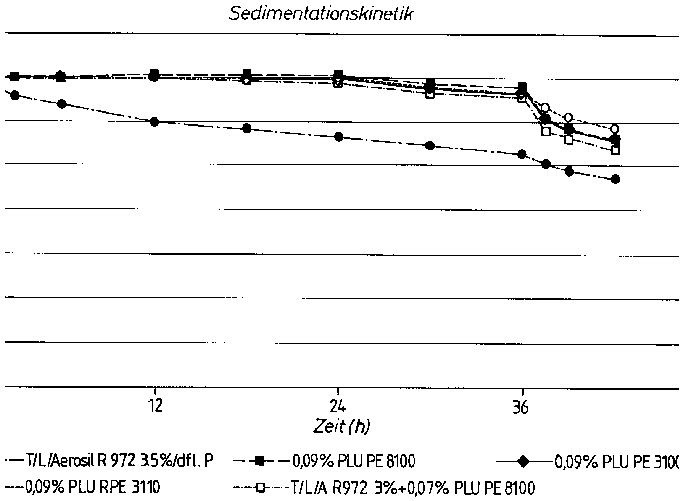

In Abbildung 1 ist die Sedimentationskinetik von Formulierungen mit hydrophober Kieselsäure dargestellt. Die Messungen wurden mit einer sogenannten Lumifuge durch Messung der Lichtbeugung durchgeführt. Die Sedimentationskinetik stellt die Sedimentation der Formulierung auf der y- Achse ("*Interphase Height")* und die Zeitdauer der Auswirkung der Schwerkraft im Zentrifugalfeld auf der x-Achse dar (angegeben in Stunden). Eine Formulierung, die eine *Interphase Height* von 100 % hat, sedimentiert nicht unter der angelegten Schwerkraft, eine Formulierung mit z.B. 80 % Interphase Height sedimentiert stärker als eine mit z.B. 90 %.

Die gemessenen Formulierungen, unter anderem die des Beispiels 13, enthalten eine hydrophobe Kieselsäure (Aerosil R972, eine methylierte Kieselsäure der Firma Degussa) und Toltrazuril in dünnflüssigem Paraffin. Die unterste Kurve enthält keinen Zusatz an polyethoxylierten Verbindungen, die anderen Kurven enthalten als Zusatz polyethoxylierte Verbindungen, und zwar Poloxamere der Firma BASF (Pluronic PE8100, Pluronic PE 3100 und Pluronic RPE3110).

Es ist zu sehen, dass die Formulierung mit hydrophober Kieselsäure ohne Zusatz von einer polyethoxylierten Verbindung (Poloxamer) den niedrigsten Kurvenverlauf aufweist. Alle anderen Formulierungen, die neben der hydrophoben Kieselsäure den Zusatz von polyethoxylierten Verbindungen (verschiedene Poloxamere wie oben benannt) enthalten, liegen deutlich höher. Damit sind die Sedimentationeigenschaften (i.e. die Verhinderung der Sedimentation) der Formulierungen mit Zusatz von polyethoxylierten Verbindungen im Vergleich zu der Formulierung ohne Zusatz von polyethoxylierten Verbindungen deutlich verbessert.

### Beispiele

Die Prozentangaben der hier beschriebenen Formulierungen sind in Gewicht pro Volumen angegeben. Als Mittelkettige Triglyceride sind die Triglyceride der Capryl- /Caprinsäureester zu verwenden, beispielsweise Miglyol® 812 der Fa. Sasol/Witten (z. B. verwendet in den Beispielen 3 und 6). Als hochdisperse, hydrophobe Kieselsäuren wird die methylierte Kieselsäure Aerosil® R972 der Fa. Degussa verwendet. (Aerosil R972 ist eine mit Dimethyldichlorsilan hydrophobisierte, pyrogene Kieselsäure, basierend auf hydrophiler pyrogener Kieselsäure mit einer spezifischen Oberfläche von ca. 130 m²/g und einen Methylierungsgrad von 66%-75%).

### Beispiel 1

0,14 % Pradofloxacin Trihydrat
1,0 % Clotrimazol
0,05 % Dexamethasonacetat
4 % Benzylalkohol
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 91 g MCT auf 60 °C erwärmt und gelöst. Bei ca. 22 ° werden 1,0 g Clotrimazol sowie 0,05 g Dexamethasonacetat suspendiert. 0,14 g Pradofloxacin Trihydrat werden in 4 g Benzylalkohol gelöst und in die Suspension gemischt. 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 2

0,14 % Pradofloxacin Trihydrat
1,0 % Clotrimazol
0,05 % Dexamethasonacetat
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 95 g MCT auf 60 °C erwärmt und gelöst. Bei ca. 22 ° werden 0,14 g Pradofloxacin Trihydrat, 1,0 g Clotrimazol, 0,05 g Dexamethasonacetat, 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 3

0,14 % Pradofloxacin Trihydrat
1,0 % Clotrimazol
0,05 % Dexamethasonacetat
4 % Benzylalkohol
0,1 % Sorbinsäure
0,05 % Glycerin-Polyethylenglykolricinoleat (hergestellt aus 1 mol Rizinusöl und 35 mol Ethylenoxid. Das Produkt enthält 83% hydrophobe Polyoxyethylenglycerolricinoleat-Verbindungen und ca. 17% hydrophile Polyoxyethylenglycerol und Polyethylenglykol)
3,2 % Hochdisperse, hydrophobe Kieselsäure
ad 100 % Mittelkettige Triglyceride
0,1 g Sorbinsäure werden in 91 g MCT auf 60 °C erwärmt und gelöst. Bei ca. 22 ° werden 1,0 g Clotrimazol sowie 0,05 g Dexamethasonacetat suspendiert. 0,14 g Pradofloxacin Trihydrat werden in 4 g Benzylalkohol gelöst und in die Suspension gemischt. 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 4

0,14 % Pradofloxacin Trihydrat
1,0 % Clotrimazol
0,05 % Dexamethasonacetat
0,1 % Sorbinsäure
0,05 % Glycerin-Polyethylenglykolricinoleat
3,2 % Hochdisperse, hydrophobe Kieselsäure
ad 100 % Mittelkettige Triglyceride
0,1 g Sorbinsäure werden in 95 g MCT auf 60 °C erwärmt und gelöst. Bei ca. 22 ° werden 0,14 g Pradofloxacin Trihydrat, 1,0 g Clotrimazol, 0,05 g Dexamethasonacetat, 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 5

0,14 % Pradofloxacin Trihydrat
1,0 % Bifonazol
0,05 % Dexamethasonacetat
4 % Benzylalkohol
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 91 g MCT auf 60 °C erwärmt und gelöst. Bei ca. 22 ° werden 1,0 g Bifonazol sowie 0,05 g Dexamethasonacetat suspendiert. 0,14 g Pradofloxacin Trihydrat werden in 4 g Benzylalkohol gelöst und in die Suspension gemischt. 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 6

0,14 % Pradofloxacin Trihydrat
1,0 % Bifonazol
0,05 % Dexamethasonacetat
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 95 g MCT auf 60 °C erwärmt und gelöst. Bei ca. 22 ° werden 0,14 g Pradofloxacin Trihydrat, 1,0 g Bifonazol, 0,05 g Dexamethasonacetat, 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 7

0,1 % Enrofloxacin
1,0 % Clotrimazol
0,05 % Dexamethasonacetat
4 % Benzylalkohol
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 91 g MCT auf 60 °C erwärmt und gelöst. Bei ca. 22 ° werden 1,0 g Clotrimazol sowie 0,05 g Dexamethasonacetat suspendiert. 0,1 g Enrofloxacin werden in 4 g Benzylalkohol gelöst und in die Suspension gemischt. 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 8

0,1 % Enrofloxacin
1,0 % Clotrimazol
0,05 % Dexamethasonacetat
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 95 g MCT auf 60 °C erwärmt und gelöst. Bei ca. 22 ° werden 0,1 g Enrofloxacin, 1,0 g Clotrimazol, 0,05 g Dexamethasonacetat, 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 9

0,1 % Enrofloxacin
1,0 % Bifonazol
0,05 % Dexamethasonacetat
4 % Benzylalkohol
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 91 g MCT auf 60 °C erwärmt und gelöst. Bei ca. 22 ° werden 1,0 g Bifonazol sowie 0,05 g Dexamethasonacetat suspendiert. 0,1 g Enrofloxacin werden in 4 g Benzylalkohol gelöst und in die Suspension gemischt. 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 10

0,1 % Enrofloxacin
1,0 % Bifonazol
0,05 % Dexamethasonacetat
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 95 g MCT auf 60 °C erwärmt und gelöst. Bei ca. 22 ° werden 0,1 g Enrofloxacin, 1,0 g Bifonazol, 0,05 g Dexamethasonacetat, 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 11

0,1 % Enrofloxacin
1,0 % Bifonazol
0,05 % Triamcinolonacetat
4 % Benzylalkohol
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 91 g MCT auf 60 °C erwärmt und gelöst. Bei ca. 22 ° werden 1,0 g Bifonazol sowie 0,05 g Triamcinolonacetat suspendiert. 0,1 g Enrofloxacin werden in 4 g Benzylalkohol gelöst und in die Suspension gemischt. 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 12

0,1 % Enrofloxacin
1,0 % Bifonazol
0,05 % Triamcinolonacetat
0,1 % Sorbinsäure
0,05 % Polyoxyethylen-20-sorbitanmonooleat
3,2 % Hochdisperse, hydrophobe Kieselsäure
ad 100 % Mittelkettige Triglyceride
0,1 g Sorbinsäure werden in 95,5 g MCT auf 60 °C erwärmt und gelöst. Bei ca. 22 ° werden 0,1 g Enrofloxacin, 1,0 g Bifonazol, 0,05 g Triamcinolonacetat, 0,05 g Polyoxyethylen-20-sorbitanmonooleat und 3,2 g hochdisperse, hydrophobe Kieselsäure werden dispergiert. Anschließend wird mit einem Homogenisator die Suspension für ca. 10 min homogenisiert.

### Beispiel 13

5 % Toltrazuril
0,07 % Poloxamer (Pluronic PE 3100)
3 % Hochdisperse, hydrophobe Kieselsäure
ad 100 % Paraffin, dünnflüssig 5 g Toltrazuril, 0,07 g Poloxamer und 3 g hochdisperse, hydrophobe Kieselsäure werden in 92 g Paraffin dispergiert. Anschließend wird mit einem Homogenisator die Suspension für ca. 10 min homogenisiert.

## Patentansprüche

1. Verwendung von amphiphilen Substanzen zur Stabilisierung von fluiden Suspensionen auf Ölbasis enthaltend eine hydrophobe Kieselsäure, in der Silanolgruppen durch Alkylgruppen alkyliert sind, wobei die amphiphile Substanz eine polyoxyethylierte Verbindung ist.

2. Verwendung gemäß Anspruch 1, wobei die fluiden Suspensionen auf Ölbasis
a) einen Wirkstoff
b) eine hydrophobe Kieselsäure, in der Silanolgruppen durch Alkylgruppen alkyliert sind und
c) eine polyoxyethylierte Verbindung enthalten.

3. Verwendung gemäß einem der Ansprüche 1 oder 2, wobei die fluiden Suspensionen auf Ölbasis als polyoxyethylierte Verbindung Poloxamere; Polyoxyethylen-Fettsäureglyceride wie Glycerin-Polyethylenglykolricinoleat; Polyoxyethylen-Sorbitanfettsäureester wie Polyoxyethylen-20-sorbitanmonooleat; Polyoxyethylen-Fettsäuren wie Macrogol-15-Hydroxystearat oder Polyoxyethylen-Fettalkohole wie Hydroxypolyethoxydodecan enthalten.

4. Verwendung gemäß Anspruch 2, wobei die fluiden Suspensionen auf Ölbasis ein Fluorchinolon enthalten.

5. Verwendung gemäß Anspruch 4, wobei die fluiden Suspensionen auf Ölbasis Enrofloxacin enthalten.

6. Verwendung gemäß Anspruch 4, wobei die fluiden Suspensionen auf Ölbasis Pradofloxacin enthalten.

7. Verwendung gemäß Anspruch 4, wobei die fluiden Suspensionen auf Ölbasis Marbofloxacin enthalten.

8. Verwendung gemäß einem der Ansprüche 2 bis 7, wobei die fluiden Suspensionen auf Ölbasis zusätzlich ein Antimykotikum enthalten.

9. Verwendung gemäß Anspruch 8, wobei die fluiden Suspensionen auf Ölbasis Clotrimazol, Miconazol oder Bifonazol enthalten.

10. Verwendung gemäß einem der Ansprüche 2 bis 9, wobei die fluiden Suspensionen auf Ölbasis zusätzlich ein Kortikoid enthalten.

11. Verwendung gemäß Anspruch 10, wobei die fluiden Suspensionen auf Ölbasis Dexamethason, Betamethason oder Triamcinolon enthalten.

12. Verwendung gemäß Anspruch 2, wobei die fluiden Suspensionen auf Ölbasis zusätzlich ein Triazin enthalten.

## Claims

1. Use of amphiphilic substances for stabilizing fluid oil-based suspensions comprising a hydrophobic silica in which silanol groups are alkylated by alkyl groups, the amphiphilic substance is a polyoxyethylated compound.

2. Use according to Claim 1, wherein the fluid oil-based suspensions comprise:
a) an active ingredient
b) a hydrophobic silica in which silanol groups are alkylated by alkyl groups and
c) a polyoxyethylated compound

3. Use according to either of Claims 1 and 2, wherein the fluid oil-based suspensions comprise poloxamers; polyoxyethylene fatty acid glycerides such as glycerol polyethylene glycol ricinoleate; polyoxyethylene sorbitan fatty acid esters such as polyoxyethylene 20 sorbitan monooleate; polyoxyethylene fatty acids such as macrogol 15 hydroxystearate or polyoxyethylene fatty alcohols such as hydroxypolyethoxydodecane, as polyoxyethylated compound.

4. Use according to Claim 2, wherein the fluid oil-based suspensions comprise a fluoroquinolone.

5. Use according to Claim 4, wherein the fluid oil-based suspensions comprise enrofloxacin.

6. Use according to Claim 4, wherein the fluid oil-based suspensions comprise pradofloxacin.

7. Use according to Claim 4, wherein the fluid oil-based suspensions comprise marbofloxacin.

8. Use according to any of Claims 2 to 7, wherein the fluid oil-based suspensions additionally comprise an antimycotic.

9. Use according to Claim 8, wherein the fluid oil-based suspensions comprise clotrimazole, miconazole or bifonazole.

10. Use according to any of Claims 2 to 9, wherein the fluid oil-based suspensions additionally comprise a corticoid.

11. Use according to Claim 10, wherein the fluid oil-based suspensions comprise dexamethasone, betamethasone or triamcinolone.

12. Use according to Claim 2, wherein the fluid oil-based suspensions additionally comprise a triazine.

## Revendications

1. Utilisation de substances amphiphiles pour la stabilisation de suspensions fluides à base d'huile, contenant une silice hydrophobe, dans laquelle les groupes silanol sont alkylés par des groupes alkyle, la substance amphiphile étant un composé polyoxyéthylé.

2. Utilisation selon la revendication 1, les suspensions fluides à base d'huile contenant
a) une substance active
b) une silice hydrophobe, dans laquelle les groupes silanol sont alkylés par des groupes alkyle et
c) un composé polyoxyéthylé.

3. Utilisation selon l'une quelconque des revendications 1 ou 2, les suspensions fluides à base d'huile contenant, comme composé polyoxyéthylé, des poloxamères ; des glycérides de polyoxyéthylène-acide gras, tels que le ricinoléate de glycérol-polyéthylèneglycol ; les esters de polyoxyéthylène-acide gras de sorbitane, tels que le monooléate de polyoxyéthylène-20-sorbitane ; les polyoxyéthylène-acides gras tels que le Macrogol-15-hydroxystéarate ou les polyoxyéthylène-alcools gras, tels que l'hydroxypolyéthoxydodécane.

4. Utilisation selon la revendication 2, les suspensions fluides à base d'huile contenant une fluoroquinolone.

5. Utilisation selon la revendication 4, les suspensions fluides à base d'huile contenant de l'enrofloxacine.

6. Utilisation selon la revendication 4, les suspensions fluides à base d'huile contenant de la pradofloxacine.

7. Utilisation selon la revendication 4, les suspensions fluides à base d'huile contenant de la marbofloxacine.

8. Utilisation selon l'une quelconque des revendications 2 à 7, les suspensions fluides à base d'huile contenant en outre un antimycosique.

9. Utilisation selon la revendication 8, les suspensions fluides à base d'huile contenant du chlotrimazole, du miconazole ou du bifonazole.

10. Utilisation selon l'une quelconque des revendications 2 à 9, les suspensions fluides à base d'huile contenant en outre un corticoïde.

11. Utilisation selon la revendication 10, les suspensions fluides à base d'huile contenant de la dexaméthasone, de la bêtaméthasone ou de la triamcinolone.

12. Utilisation selon la revendication 2, les suspensions fluides à base d'huile contenant en outre une triazine.
